Europäisches Patentamt

European Patent Office

Office européen des brevets

(11) Publication number: **0 000 556**
B1

# EUROPEAN PATENT SPECIFICATION

(21) Application number: 78100459.3

(22) Date of filing: 20.07.78

(51) Int. Cl.³: **A 61 B 5/05,** A 61 N 1/36,
A 61 C 19/04

(54) Testing device for testing the dental pulp of a tooth.

(30) Priority: 25.07.77 US 818737

(43) Date of publication of application:
07.02.79 Bulletin 79/3

(45) Publication of the grant of the European patent:
11.03.81 Bulletin 81/10

(84) Designated Contracting States:
CH DE FR GB SE

(56) References cited:
CH - A - 572 346
US - A - 3 128 759
US - A - 3 768 017
US - A - 3 894 532

BIOMEDICAL ENGINEERING, vol. 11, no. 1,
January 1976, London GB
J.M. MUMFORD & D.G. LEWIS: "Electronic tooth
stimulator for pain research", pages 22 + 23

(73) Proprietor: Masreliez, C. Johan
3301 181 st Place N.E.
Redmond Washington 98052 (US)

(72) Inventor: Masreliez, C. Johan
3301 181 st Place N.E.
Redmond Washington 98052 (US)

(74) Representative: Patentanwälte Dipl.-Ing. A.
Grünecker, Dr.-Ing. H. Kinkeldey, Dr.-Ing. W.
Stockmair,
Dr. rer. nat. K. Schumann, Dipl.-Ing. P. Jakob, Dr.
rer. nat. G. Bezold Maximilianstrasse 43
D-8000 München 22 (DE)

Courier Press, Leamington Spa, England.

## Testing device for testing the dental pulp of a tooth

The invention relates to a testing device for testing the dental pulp of a tooth as referred to in the introductory part of claim 1.

When a tooth has been subject to decay, physical trauma, thermal changes or irritation by sweet foods or deep fillings, the pulp tissue becomes inflamed. When this inflammation is left untreated for a long time, the ensuing pulpal inflammation generally proceeds to a severe infection with abscess formation about the root tips. It is at this stage that many teeth require extraction and that endodontic procedures, designed to help remove this infection, are least successful.

Electric devices used to test the vitality of pulpal tissue have long been used as diagnostic aids by dentists. For example, these devices have found application where a patient complains of pain on one side of the mouth and cannot isolate the specific tooth from which the pain emanates. Also they have been used in the diagnosis of decay which develops around the borders of fillings in teeth with existing restorations, a situation where X-rays alone may not indicate the presence or extent of the decay.

In actual operation, a stimulating electrode of an electric pulp tester is applied to a tooth which is undergoing examination. The level of the voltage at the electrode is then gradually raised until the patient feels a tingling or mild electric shock in the tooth that is being touched by the electrode. By comparing the voltage level to which the patient responds with the level at which other of his normal teeth respond, the dentist can make a diagnosis on the state of inflammation or vitality of the dental pulp in the tooth under examination.

While the existing dental pulp testers have proved to be valuable diagnostic tools, they suffer from certain disadvantages which have limited their utility. A disadvantage of conventional pulp testers is that a reasonable testing rate can only be achieved by rapidly increasing the intensity of the stimulation. However, this rapid increase causes the intensity to "overshoot" the point where the patient can perceive the stimulation before the probe can be removed from the tooth and the increase in intensity can be terminated. Consequently, the patient is subjected to unnecessary pain, and the intensity readings taken from the display are erroneously high.

Still another disadvantage of conventional pulp testers is the characteristics of the electrical stimulus which they apply to a tooth. The electrical stimulus is generally either a continuous voltage having a magnitude which increases with time, or a continuous series of pulses having an amplitude which increases with time. A testing device of the last-mentioned type is known from Biomedical Engineering, Vol. 11, No. 1, January, 1976, J.M. Mumford and D.G. Lewis "Electronic Tooth Stimulator for Pain Research", pages 22 and 23. The probe used in this device includes a microswitch which allows the amplitude of the pulses applied to the electrode to increase as long as it is pressed. Actuation of this microswitch, however, directs the attention of the dental practitioner away from the patient. A more serious disadvantage of this known device, however, is that by constantly stimulating the tooth, it loses part of its sensitivity.

The object underlying the invention is to provide for a testing device of the kind mentioned in the introductory part of claim 1, being easier to handle by the practitioner and leading to more reliable medical results.

This task is performed by the features being included in the characterizing clause of claim 1.

The testing device according to the invention has a contact detector for sensing when the electrode of the probe makes contact with the tooth under test. The contact detector causes the intensity of the stimulus to increase from a low initial level until the probe is removed from the tooth. The intensity of the stimulus when the probe is removed from the tooth is then displayed on a digital display. When the probe once again makes contact with a tooth the display is reset to the initial value of intensity, and the intensity of the stimulus increases from the initial value. The stimulation is in the form of intermittently produced bursts of pulses with the amplitude of each burst being larger than the amplitude of the previous burst. Consequently, the dental pulp is allowed to reset before each increase in the intensity of the stimulus.

Further preferred embodiments are claimed in claims 2-11.

The invention is described in more detail in the following description of the drawings, wherein

Fig. 1 is an isometric view of the dental pulp tester in use;

Fig. 2 is a schematic of a first embodiment of the dental pulp tester;

Fig. 3 is a cross-sectional view of the dental probe of the dental pulp tester;

Fig. 4 is a cross-sectional view taken along the line 4—4 of Fig. 3, and

Fig. 5 is a schematic of a part of second embodiment of the dental pulp tester.

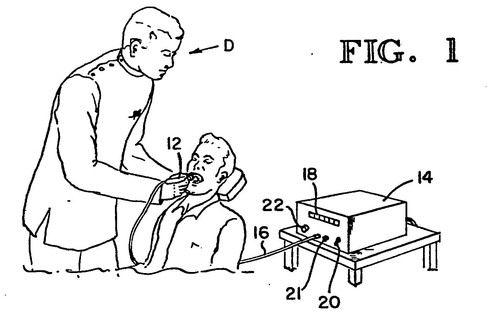

The dental pulp tester is used to test the dental pulp in the teeth of a patient P as illustrated in Fig. 1. The dental practitioner D utilizes a probe 12 having an electrode contacting a tooth under test. The probe 12 is connected to a testing unit 14 through electric conductors 16. The testing unit 14 includes a digital display 18 which provides an indication of the intensity of the electrical stimulus from the probe 12. The only controls and indicators for the unit are an on-off switch 20, a low-voltage warning light 21 which is illuminated if the amplitude of the electrical stimulus falls below a minimum value, and a sweep rate control 22 which adjusts the rate at which the intensity of the stimulus increases. Both of these controls 20, 22 normally remain stationary while the dental pulp of a patient P is being tested.

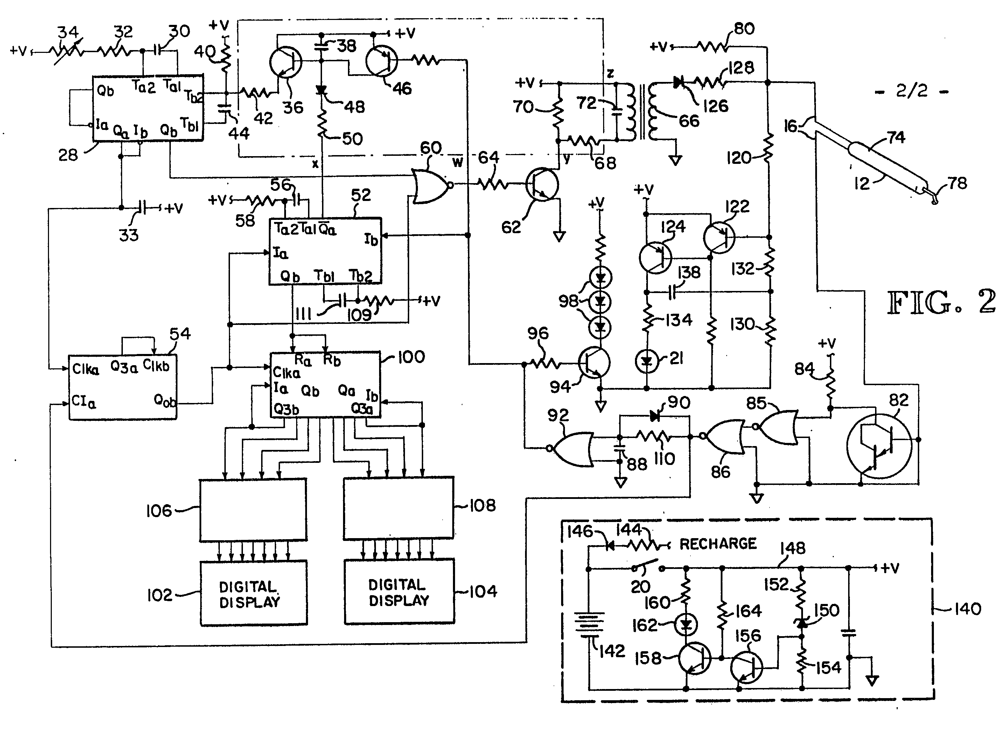

As illustrated in Fig. 2, the electrical stimulus originates in a pulse generator 28 having a pulse width determined by a pulse width control voltage. The voltage controlled pulse generator 28 may be implemented by an integrated circuit dual monostable multi-vibrator or "one-shot" such as a Fairchild F4528 or Motorola MC14528. Basically, the circuit consists of two one-shots 28a,b each having an output $Q_a$, $Q_b$ triggering the input $I_{b,a}$ of the other so that the circuits are periodically triggered. The first one-shots 28a generates pulses having a manually adjustable duration, while the pulses generated by the second one-shot 28b have a duration determined by a control input. The duration of the pulses from the first one-shot 28a is determined by timing capacitor 30 and the series combination of fixed resistor 32 and variable resistor 34 which is adjusted, as explained hereinafter, to control the rate at which the intensity of the electrical stimulation is increased. A capacitor 33 is connected between supply voltage and the input $I_b$ of the second one-shot in order to trigger the second one-shot 28b when power is applied to the unit.

The duration of the pulses produced by the second one-shot 28b is determined by the voltage at the base of transistor 36. Transistor 36 acts as a voltage follower to provide a high impedance discharge path from capacitor 38 to the pulse generator 28. Resistors 40, 42 and capacitor 44 are provided to bias the pulse duration at a predetermined value. The capacitor 38 is initially discharged by transistor 46 at the start of each test. Thereafter the capacitor 38 is charged through diode 48 and resistor 50 by periodic, negative going pulses from the $Q_a$ output of a first one-shot 52a in a dual one-shot circuit 52. The first one-shot 52a is triggered at $I_a$ by the $Q_{ob}$ output of decade counters 54b which is driven by the $Q_{3a}$ output of decade counter 54a in series driven by the pulse generator 28. Consequently, the capacitor 38 is incrementally charged for each 20 pulses from pulse generator 28. The duration of the pulse from the one-shot 52, and hence the amount of charge provided during each increment, is determined by timing capacitor 56 and resistor 58.

The $Q_{ob}$ output of the counter 54b also gates the output of the pulse generator 28 through NOR gate 60 to the base of a transistor 62 through resistor 64. Since the output $Q_{ob}$ is alternately low for 10 pulses of pulse generator 28 and then high for 10 pulses of pulse generator 28, NOR gate 60 gates 10 pulses to the transistor 62 and then cuts off the transistor 62 for 10 pulses. Consequently, a "dead space" is produced after each burst of 10 pulses which, as explained hereinafter, allows the nerves in the dental pulp to reset after each stimulus so that the nerves are at the maximum sensitivity at the start of each stimulus. As the transistor 62 saturates, current flows through the primary of transformer 66 and resistor 68. Resistor 68 is selected to limit the maximum current flow through the primary of transformer 66. The pulses across the secondary of the transformer, which are generated inductively when turning off the transistor 62, have a peak amplitude which is determined by the value of resistor 70 and the duration of the pulses at the output of the NOR gate 60. Capacitor 72 is provided to dampen the reverse emf spike generated when current flow through transistor 62 is terminated.

In summary, 10 pulses from the pulse generator 28 are gated through the NOR gate 60 to produce 10 equal amplitude pulses across the secondary of transformer 66 followed by a dead space of 10 pulses during which the NOR gate 60 is gated off. After each pulse burst of 10 pulses, the $I_a$ input of one-shot 52a is triggered to generate a charging pulse on the $Q_a$ output of one-shot 52 which incrementally charges capacitor 38 and decreases the control voltage to the pulse generator 28 so that the subsequent burst of pulses from the NOR gate 60 has an increased duration resulting in pulses of increased amplitude across the secondary of the transformer 66. The rate at which the electrical stimulus increases can be varied by adjusting the sweep rate resistor 34 which is controlled by the sweep rate control 22 on the front panel of the unit 14 (Fig. 1).

As explained in greater detail hereinafter, the electrode 74 includes a conductive outer sleeve 74 surrounding, and insulated from, and elongated center electrode 78. In operation the center electrode 78 makes contact with the tooth of a patient, and the electrode 74 is in electrical contact with the patient through the dentist's hand and the patient's lip. The electrode 74 is connected to the power supply output through resistor 80 so that when the electrode 78 makes contact with the tooth, current flows through resistor 80 and probe 12 into the base of the darlington pair 82. As the darlington pair 82 becomes saturated the current flowing through resistor 84 causes the voltage at the collector of the darlington pair 82 to go low thereby causing the output of NOR gate 85 to go high and the output of NOR gate 86 to go low. The output of NOR gate 86 is connected to the counter inhibit input $Cl_a$ of a counter 54a so that as the output of NOR gate 86 goes low counter 54 begins incrementing. At the same time capacitor 88 is discharged through diode 90 so that the output of NOR gate 92 goes high. NOR gate 92 then saturates transistor 94 through resistor 96 and illuminates three light emitting diodes 98 which, as explained hereinafter, are visibly mounted on the probe 12. NOR gate 92 also turns off transistor 46 permitting capacitor 38 to be charged, and it triggers the $I_b$ input of one-shot 52b thereby producing a pulse at the $Q_b$ output which resets counter 100. Counter 100 counts the pulses from the output of counter 54 and displays the contents on digital displays 102, 104 through BCD-to-7 segment latch/decoder/drivers 106, 108, respectively. The outputs of the counter 100 are continuously displayed until the counter 100 is reset by one-shot 52b. the input $I_b$ of one-shot 52b is connected to the output of NOR gate 92 so that a reset pulse is generated at the output $Q_b$ of one-shot 52b when the output of NOR gate 92 goes high as the probe electrode 78 makes contact with a tooth. The duration of the reset pulse, which is not critical, is determined by the values of timing resistor 109 and timing capacitor 111. The most significant bit output $Q_{3b}$ of the counter 100b is connected to the inhibit input $I_a$ of counter 100 so the counter 100 "locks up" if incremented to near its maximum capacity.

The tester unit also includes a circuit for insuring that the output voltage from the probe does not fall below a predetermined minimum level. This circuit is connected to electrode 74 through resistor 120. Transistor 122 is normally saturated thereby cutting off transistor 124. When the positive going pulse from the transformer 66 exceeds a level set by the voltage divider formed by resistors 130, 132 in combination with resistor 120, transistor 122 becomes cut off thereby saturating transistor 124 and allowing current to flow in the emitter-collector circuit through resistor 134 and light emitting diode 21. Positive feedback capacitor 138 is provided to completely saturate transistor 124 as transistor 122 goes into cutoff. Consequently, pulsating illumination from light emitting diode 21 indicates that the amplitude of the signal at the output of the transformer 66 is sufficient.

An alternative embodiment of the circuit for generating the electrical stimulus is illustrated in Fig. 5. Basically, the alternative embodiment places a linearly increasing voltage on one lead of the secondary of transformer 66 while the other lead of the transformer secondary is periodically connected to ground through transistor 62 by constant duration pulses from pulse generator 28. The embodiment illustrated in Fig. 5 is placed in the circuit of Fig. 2 with the alphabetical markings of the broken leads matching the correspondingly marked leads illustrated in Fig. 2. The negative going pulses at the $\overline{Q}_a$ output of one-shot 52 drives transistor 200 into conduction. The current through transistor 200 is proportional to the voltage across resistor 202 divided by the resistance of resistor 202. Resistors 204, 206 form a voltage divider and are selected to place the proper voltage on the emitter of transistor 200 to achieve a predetermined constant current for charging capacitor 208. As capacitor 208 continues to charge, a linearly increasing voltage is produced across capacitor 208 which is coupled to output line z by emitter follower transistor 210. Capacitor 211 is provided to filter the output line z which is secondary of transformer 66. Capacitor 208 is reset through diode 212 by a low level output of NOR gate 92 each time the electrode 78 breaks the contact with a tooth. Diode 214 is placed across the secondary of transformer 66 to dampen reverse emf transients which are produced when current through the transistor 62 is terminated. In this alternate embodiment, the probe stimulus output pulses are generated when turning on transistor 62 rather than as in the previous embodiment when turning off transistor.

The dental pulp tester unit also includes an internal power supply 140 having a rechargeable battery 142 which may be recharged through resistor 144 and rectifying diode 146. When the on-off switch 20 is in its on position the battery 142 is connected to the power supply line 148. The power supply 140 includes a low-voltage warning circuit which indicates when the battery 142 must be recharged. As long as the voltage on line 148 exceeds the reverse breakdown voltage of zener diode 150 current flows through resistors 152, 154. Under these conditions, transistor 156 is saturated thereby cutting off transistor 158 so that current is unable to flow through resistor 160 and light emitting diode 162. When the voltage on line 148 drops below the breakdown voltage of zener diode 150, transistor 156 is cut off thereby allowing current to flow through resistor 164 and the base-emitter junction of transistor 158. Transistor 158 then saturates allowing current to flow through light emitting diode 162 and indicate that the battery 142 is in need of recharging.

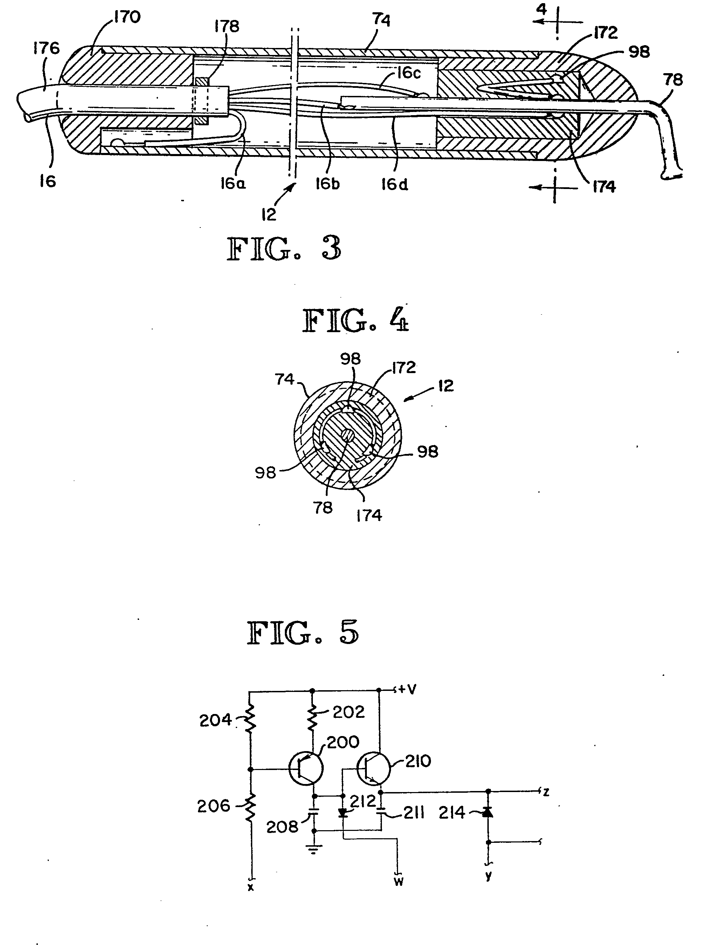

The structure of the probe 12 is best illustrated in Figs. 3 and 4. The probe 12 includes a cylindrical conductor forming the outer electrode 75 having its ends closed by a pair of end caps 170, 172. The end cap 172 is formed of an insulative, light-transmissive substance such as plastic, and it contains an axial bore which receives the electrode 78 which makes contact with the tooth. A transparent, cylindrical insert 174 placed in a bore formed in the end cap 172 contains the three light emitting diodes 98 which indicate that the electrode 78 has made contact with a tooth as described above. The cable 16 includes a first conductor 16a connected to the outer electrode 74, a second conductor 16b connected to the inner electrodes 78 and a pair of conductors 16c,d completing a circuit with the light emitting diodes 98. The conductors 16 are encased in a cylindrical sheathing 176 which passes through a bore in the end cap 170 and is retained in place by an annular washer 178.

In operation, when the electrode 78 of the probe 12 first makes contact with a tooth the output of NOR gate 86 goes low thereby illuminating light emitting diodes 98, releasing integrating capacitor 38, resetting counter 100 and allowing counter 54 to begin incrementing. When the output of counter 54 goes low the pulses from the output of the pulse generator 28 are gated through NOR gate 60 to drive transformer 66 and generate 10 pulses at the probe 12. The output of counter 54 then goes high thereby gating NOR gate 60 off and triggering a pulse from the $\bar{Q}_a$ output of one-shot 52a which incrementally charges integrating capacitor 38. When the output of counter 54 again goes high, pulses having an increased duration are gated through NOR gate 60 so that the amplitude of the pulses across the secondary transformer 60 are increased. The pulse bursts continue to increase in amplitude until the electrical stimulus is felt by the patient at which time the electrode 78 is removed from the tooth of the patient. Since the amplitude of the pulses in each pulse burst is incrementally increased after every 20 pulses from the pulse generator 28, the number of pulses counted by counter 100, as indicated by display 102, 104, is an indication of the amplitude of the pulses delivered to the probe 12. When the electrode 78 is removed from the tooth the current path through the probe 12 is broken so that the output of NOR gate 86 goes high thereby charging capacitor 88 through resistor 110. After a predetermined delay time, the output of NOR gate 92 goes low thereby resetting capacitor 38 and extinguishing the light emitting diodes 98. The time delay provided by capacitor 88 and resistor 110 prevents the counter 100 and integrating capacitor 38 from becoming reset should the electrode 78 of the probe momentarily lose contact with the tooth. However, loss of contact between the electrode 78 and the tooth will inhibit the counter 54 to prevent the counter 100 from incrementing and the duration of the pulses from the pulse generator 28 from increasing.

## Claims

1. Testing device for testing the dental pulp of a tooth, comprising,

a probe (12) having a first electrode (78) adapted to directly contact the tooth,

a second electrode (74) adapted for indirect electrical contact with the tooth,

an electrical stimulus circuitry (28, 30, 36, 38, 52, 60, 62, 66) connected to the probe (12) and producing an electrical stimulus between the first and second electrodes (78, 74) *having an intensity which increases with time* responsive to an initiate signal,

a display (102, 104) providing an indication of the intensity of the electrical stimulus and retaining the indication of electrical stimulus intensity subsequent to termination of the initiate signal such that the display continually indicates the maximum value of electrical stimulus,

a contact decoder (82, 98) for determining when the first electrode (78) is in contact with the tooth and for generating the initiate signal in response thereto such that the intensity of the electrical stimulus is automatically increased responsive to the first electrode (78) contacting the tooth,

characterized in that

the stimulus circuitry (28, 30, 36, 38, 52, 60, 62, 66) provides repetitively generated bursts of pulsating signals, the amplitude of the signals being relatively constant during each burst and the amplitude of the signals during each burst being greater than the amplitude of signals during the previous burst,

the contact detector resets the intensity of the electrical stimulus to a predetermined value responsive to termination of the initiate signal for longer than a predetermined period such that the intensity of the electrical stimulus is initialized to a preset value by removal of the first electrode (78) from the tooth for a longer than a predetermined period, the display (102, 104) is operatively associated with the stimulus circuitry and retains the indication of electrical stimulus intensity subsequent to termination of the initiate signal and automatically removes the indication of electrical stimulus intensity and resets the *indication* of the electrical stimulus to a predetermined value upon subsequent commencement of the initiate signal.

2. Testing device as claimed in claim 1, characterized in that, the contact detector (82, 98) is operable independently from the stimulus circuitry (28, 30, 36, 38, 52, 60, 62, 66), such that the contact detector may be actuated prior to generation of the stimulus.

3. Testing device as claimed in claims 1 or 2, characterized in that, the rate at which the intensity of the stimulus increases is adjustable.

4. Testing device as claimed in one of claims 1—3, characterized in that there is provided a counter (100) for counting the number of pulses generated by a signal generator (28) during the period the initiate signal is being produced and, a display (102, 104) for displaying the contents of the counter.

5. Testing device as claimed in claim 4, characterized in that the signal generator (28) is provided to maintain the duration of the pulses constant responsive to termination of the initiate signal for less than a predetermined period such that the intensity of the electric stimulus remains constant when the first electrode (78) loses contact with the tooth for less than a predetermined period.

6. Testing devices at least as claimed in claim 4, characterized in that the counter (100) is reset to an initial value at the commencement of the initiate signal such that the digital display (102, 104) indicates the maximum value of the electrical stimulus after the first electrode (78) is removed from the tooth until the first electrode subsequently contacts the tooth.

7. Testing device as claimed in one of claims 1—6, characterized in that the probe (12) includes a contact indicator (98) for producing a visual indication of the initiate signal thereby producing a visual indication of electrical contact with the tooth.

8. Testing device as claimed in one of claims 4—7, characterized in that the signal generator (28) is voltage controlled and there is provided a capacitor (38) having a pair of electrical contacts, one of which is maintained at a fixed potential, a timer (30, 32) triggered by the pulses from the voltage controlled signal generator (28) for periodically generating a stimulus increase signal in response thereto, and an electrical circuitry (46) to charge on the capacitor responsive to the stimulus increase signal such that the voltage across the capacitor incrementally varies with time thereby generating the pulse control signal for the voltage controlled pulse generator (28) on the other of the capacitor contacts.

9. Testing device as claimed in one of claims 4—8, characterized in that there is provided a transformer (66) having its primary driven by the signal generator (28), and its secondary connected between the first and second electrodes (78, 74), the transformer having a relatively slow response time such that the transformer remains unsaturated when receiving the longest duration of the pulses, such that the magnitude of the signal across the secondary is proportional to the width of the pulses.

10. Testing device as claimed in claim 9, characterized in that *a voltage which is proportional to the pulse control signal is applied* to the primary of the transformer (66) for allowing current to flow through the primary responsive to pulses from the pulse generator (28) such that the pulses are generated across the electrodes (78, 74) having an amplitude proportional to the amplitude of the pulse control signal and a frequency corresponding to the frequency of the pulses from the pulse generator.

11. Testing device as claimed in one of claims 4—10, characterized in that there is provided a gate circuitry (60) for intermittently transmitting the pulses of the signal generator (28) such that the electrical stimulation is produced in intermittent bursts of pulsating signals thereby allowing the pulpal tooth nerve to reset between the bursts.

**Patentansprüche**

1. Prüfeinrichtung zum Prüfen der Zahnpulpa eines Zahnes bestehend aus

einer Sonde (12) mit einer ersten Elektrode (78), die zum direkten Kontakt mit dem Zahn angepaßt ist,

einer zweiten Elektrode (74), die zum indirekten elektrischen Kontakt mit dem Zahn angepaßt ist,

einem elektrischen Stimulusschaltkreis (28, 30, 36, 38, 52, 60, 62, 66), der mit der Sonde (12) verbunden ist und einen elektrischen Stimulus zwischen der ersten und der zweiten Elektrode (78, 74) mit einer Intensität erzeugt, welche auf ein Anfangssignal reagierend mit der Zeit zunimmt,

einer Anzeige (102, 104), die eine Anzeige der Intensität des elektrischen Stimulus liefert und die Anzeige der elektrischen Stimulusintensität anschließend an das Aufhören des Anfangssignals aufrechterhält, so daß die Anzeige fortlaufend den maximalen Wert des elektrischen Stimulus anzeigt, und

einen Kontaktfühler (82, 98) um zu bestimmen, wenn sich die erste Elektrode (78) mit dem Zahn in Kontakt befindet, und um in Abhängigkeit davon das Anfangssignal zu erzeugen, so daß die Intensität des elektrischen Stimulus automatisch in Abhängigkeit von der Kontaktherstellung der ersten Elektrode (78) mit dem Zahn erhöht wird,

dadurch gekennzeichnet,

daß der Stimulusschaltkreis (28, 30, 36, 38, 52, 60, 62, 66) wiederholt Impulssignalstöße erzeugt, wobei die Signalamplituden während jedes Signalstoßes relativ konstant sind und die Signalamplituden während jedes Signalstoßes größer als die Signalamplituden während des vorhergehenden Signalstoßes sind,

daß der Kontaktfühler die Intensität des elektrischen Stimulus beim Aufhören des Anfangssignals während einer längeren Zeit als ein vorbestimmtes Zeitintervall auf einen vorbestimmten Wert wiedereinstellt, so daß die Intensität des elektrischen Stimulus durch Entfernen der ersten Elektrode (78) von dem Zahn während einer längeren Zeit als ein vorbestimmtes Zeitintervall auf einen vorbestimmten Wert wiedereingestellt wird, und

daß die Anzeige (102, 104) betriebsmäßig dem Stimulusschaltkreis zugeordnet ist und daß die Anzeige der elektrischen Stimulusintensität anschließend an das Aufhören des Anfangssignals beibehält und automatisch die Anzeige der elektrischen Stimulansintensität entfernt und die Anzeige des elektrischen Stimulus beim folgenden Beginn des Anfangssignals auf einen vorbestimmten Wert zurücksetzt.

2. Prüfeinrichtung nach Anspruch 1, dadurch gekennzeichnet, daß der Kontaktfühler (82, 98) unabhängig, von dem Stimulusschaltkreis (28, 30, 36, 38, 52, 60, 62, 66) betreibbar ist, so daß der Kontaktfühler vor der Erzeugung des Stimulus betätigbar ist.

3. Prüfeinrichtung nach Anspruch 1 oder 2, dadurch gekennzeichnet, daß die Geschwindigkeit mit der die Intensität des Stimulus ansteigt einstellbar ist.

4. Prüfeinrichtung nach einem der Ansprüche 1 bis 3, dadurch gekennzeichnet, daß ein Zähler (100) zum Zählen der Anzahl der von dem Signalgenerator (28) während der Zeit, während der das Anfangssignal erzeugt wird, abgegebenen Impulse und eine Anzeige (102, 104) zum Anzeigen der Zählerinhalte vorgesehen sind.

5. Prüfeinrichtung nach Anspruch 4, dadurch gekennzeichnet, daß der Signalgenerator (28) vorgesehen ist, um die Dauer der Impulse in Abhängigkeit von dem Aufhören des Anfangssignals während eines kleineren als ein vorgegebenes Zeitintervall konstant zu halten, so daß die Intensität des elektrischen Stimulus konstant bleibt, wenn die erste Elektrode (78) mit dem Zahn während eines kleineren als ein vorgegebenes Zeitintervall außer Kontakt kommt.

6. Prüfeinrichtung mindestens nach Anspruch 4, dadurch gekennzeichnet, daß der Zähler (100) zu Beginn des Anfangssignals auf einen Anfangswert zurückgesetzt wird, so daß die digitale Anzeige (102, 104) den maximalen Wert des elektrischen Stimulus anzeigt, nachdem die erste Elektrode (78) von dem Zahn entfernt worden ist und bis die erste Elektrode anschließend den Zahn berührt.

7. Prüfeinrichtung nach einem der Ansprüche 1 bis 6, dadurch gekennzeichnet, daß die Sonde (12) einen Kontaktanzeiger (98) enthält, um eine Sichtanzeige des Anfangssignals zu erzeugen, wodurch eine Sichtanzeige des elektrischen Kontakts mit dem Zahn erzeugbar ist.

8. Prüfeinrichtung nach einem der Ansprüche 4 bis 7, dadurch gekennzeichnet, daß der Signalgenerator (28) spannungsgesteuert ist, und daß vorgesehen sind ein Kondensator (38) mit einem Paar elektrischer Anschlüße, von denen einer auf einem festen Potential gehalten ist, ein Zeitgeber (30, 32), welcher durch die Impulse von dem spannungsgesteuerten Signalgenerator (28) ausgelöst wird, um periodisch in Abhängigkeit davon ein Stimuluserhöhungssignal zu erzeugen, und eine elektrische Schaltungsanordnung (46), um den Kondensator in Abhängigkeit von dem Stimuluserhöhungssignal aufzuladen, so daß sich die Spannung über den Kondensator schrittweise mit der Zeit ändert, wodurch das Impulssteuersignal für den spannungsgesteuerten Impulsgenerator (28) an dem anderen Kondensatoranschluß erzeugbar ist.

9. Prüfeinrichtung nach einem der Ansprüche 4 bis 8, dadurch gekennzeichnet, daß ein Transformator (66) vorgesehen ist, dessen Primärwindung von dem Signalgenerator (28) betrieben wird und dessen Sekundärwindung zwischen der ersten und der zweiten Elektrode (78, 74) verbunden ist, wobei der Transformator eine relativ langsame Ansprechzeit aufweist, so daß der Transformator ungesättigt bleibt, wenn er die längste Dauer der Impulse erhält, so daß die Größe des Signals über die Sekundärwicklung der Breite der Impulse proportional ist.

10. Prüfeinrichtung nach Anspruch 9, dadurch gekennzeichnet, daß eine dem Impulssteuersignal proportionale Spannung an die Primärwicklung des Transformators (66) gelegt wird, damit in Abhängigkeit von den Impulsen des Impulsgenerators (28) ein Strom durch die Primärwicklung fließt, so daß die zwischen den Elektroden (78, 74) erzeugten Impulse eine der Amplitude des Impulssteuersignals proportionale Amplitude und eine der Frequenz der Impulse von dem Impulsgenerator entsprechende Frequenz aufweisen.

11. Prüeinrichtung nach einem der Ansprüche 4 bis 10, dadurch gekennzeichnet, daß ein Torschaltkreis (60) vorgesehen ist, um absatzweise Impulse von dem Signalgenerator (28) hindurchzulassen, so daß die elektrische Stimulation mit unterbrochenen Impulssignalstößen erzeugt wird, wodurch sich die Nerven der Zahnpulpa zwischen den Signalstößen beruhigen können.

**Revendications**

1. Dispositif de contrôle de l'état de la pulpe dentaire, comportant une sonde (12) possédant une première électrode (78) desintée à venir directement au contact de la dent, une seconde électrode (74) destinée à un contact électrique indirect avec la dent, un circuit d'excitation électrique (28, 30, 36, 38, 52, 60, 62, 66) relié à la sonde (12) et produisant une excitation électrique entre les première et seconde électrodes (78, 74) ayant une intensité croissant avec le temps en réponse à un signal de déclenchement, un dispositif d'affichage (102, 104) fournissant une indication de l'intensité de l'excitation électrique et conservant l'indication de l'intensité d'excitation électrique après la fin du signal de déclenchement de telle sorte que le dispositif d'affichage indique en permanence la valeur maximale de l'excitation électrique, un détecteur de contact (82, 98) pour déterminer lorsque la première électrode (78) est au contact de la dent et pour engendrer le signal de déclenchement en réponse à celui-ci de telle sorte que l'intensité de l'excitation électrique est automatiquement augmentée en réponse au contact de la première électrode (78) avec la dent, caractérisé en ce que le circuit d'excitation (28, 30, 36, 52, 60, 62, 66) délivre de façon répétitive des rafales de signaux pulsatoires, l'amplitude des signaux étant relativement constante durant chaque rafale et l'amplitude des signaux durant chaque rafale étant supérieure à l'amplitude des signaux durant la rafale antérieure, que le détecteur de contact ramène l'intensité de l'excitation électrique à une valeur prédéterminée en réponse à la suppression du signal de déclenchement pendant une période plus longue qu'une période prédéterminée de telle sorte que l'intensité de l'excitation électrique soit déclenchée à une valeur prédéterminée par retrait de la première électrode (78) de la dent pendant une durée supérieure à une durée prédéterminée, et que le dispositif d'affichage (102, 104) est associé opérativement au circuit d'excitation et conserve l'indication de l'intensité d'excitation électrique á la suite de la suppression du signal de déclenchement, et qu'il efface automatiquement l'indication de l'intensité d'excitation électrique et rétablit l'indication de l'excitation électrique à une valeur prédéterminée lors du début ultérieur du signal de déclenchement.

2. Dispositif de contrôle selon la revendication 1, caractérisé en ce que le détecteur de contact (82, 98) est actionnable indépendamment du circuit d'excitation (28, 30, 36, 38, 52, 60, 62, 66), si bien que le détecteur de contact peut être actionné avant la génération de l'excitation.

3. Dispositif de contrôle selon la revendication 1 ou 2, caractérisé en ce que la vitesse à laquelle l'intensité de l'excitation augmente est réglable.

4. Dispositif de contrôle selon une des revendications 1 à 3, caractérisé en ce qu'il est prévu un compteur (100) pour compter le nombre d'impulsions engendrées par un générateur de signaux (28) durant la période où le signal de déclenchement est produit et un dispositif d'affichage (102, 104) pour afficher le contenu du compteur.

5. Dispositif de contrôle selon la revendication 4, caractérisé en ce que le générateur de signaux (28) est prévu pour maintenir la durée des impulsions constante en réponse à une suppression du signal de déclenchement pendant moins qu'une durée prédéterminée de telle sorte que l'intensité de l'excitation électrique demeure constante lorsque la première électrode (78) perd le contact d'avec la dent pendant moins qu'une période prédéterminée.

6. Dispositif de contrôle selon la revendication 4, caractérisé en ce que le compteur (100) est restauré à une valeur initiale au début du signal de déclenchement de telle sorte que l'affichage numérique (102, 104) indique la valeur maximale de l'excitation électrique après que la première électrode (78) ait été retirée de la dent jusqu'à ce que la première électrode vienne ultérieurement au contact de celle-ci.

7. Dispositif de contrôle selon une des revendications 1 à 6, caractérisé en ce que la sonde (12) contient un indicateur de contact (98) pour fournir une indication visuelle du signal de déclenchement délivrant ainsi une indication visuelle du contact électrique avec la dent.

8. Dispositif de contrôle selon une des revendications 4 à 7, caractérisé en ce que le générateur de signaux (28) est commandé en tension et qu'un condensateur (38) est prévu, possédant une paire de contacts électriques, dont l'un est maintenu à un potentiel fixe, qu'une minuterie (30, 32) est déclenchée par les impulsions provenant du générateur de signaux commandés en tension (28) pour engendrer périodiquement un signal d'augmentation en réponse à celui-ci, et qu'un circuit électrique (46) charge le condensateur en réponse au signal d'augmentation d'excitation de telle sorte que la tension aux bornes du condensateur varie de façon incrémentielle avec le temps, engendrant ainsi le signal de contrôle d'impulsion pour le générateur d'impulsions commandé en tension (28) sur l'autre des contacts du condensateur.

9. Dispositif de contrôle selon une des revendications 4 à 8, caractérisé en ce qu'il est prévu un transformateur (66) ayant son primaire alimenté par le générateur de signaux (28), et son secondaire connecté entre les première et seconde électrodes (78, 74), le transformateur ayant un temps de réponse relativement lent de telle sorte qu'il demeure non saturé lorsqu'il reçoit la durée la plus longue d'impulsion, de telle sorte que l'amplitude du signal aux bornes du secondaire soit proportionnelle à la largeur des impulsions.

10. Dispositif de contrôle selon la revendication 9, caractérisé en ce qu'une tension proportionnelle au signal de commande d'impulsion est appliquée au primaire du transformateur (66) pour permettre une circulation de courant à travers le primaire en réponse à des impulsions provenant du générateur d'impulsions (28) de telle sorte que des impulsions sont engendrées aux bornes des électrodes (78, 74) avec une amplitude proportionnelle à l'amplitude du signal de commande d'impulsion et une fréquence correspondant à la fréquence des impulsions provenant du générateur d'impulsions.

11. Dispositif de contrôle selon une des revendications 4 à 10, caractérisé en ce qu'il est prévu un circuit porte (60) pour transmettre par intermittences les impulsions du générateur de signaux (28) de telle sorte que l'excitation électrique soit fournie en rafales intermittentes de signaux pulsatoires permettant ainsi aux nerfs de la pulpe dentaire d'être désexcités entre les rafales.

0 000 556

FIG. 1

1

FIG. 2

- 2/2 -

0000 556

FIG. 3

FIG. 4

FIG. 5